(19) Europäisches Patentamt
European Patent Office
Office européen des brevets

(11) Numéro de publication: **0 008 256**
**A2**

(12) # DEMANDE DE BREVET EUROPEEN

(21) Numéro de dépôt: **79400500.9**

(22) Date de dépôt: **13.07.79**

(51) Int. Cl.[3]: **C 07 D 295/08**, C 07 C 69/65, A 61 K 31/495

(30) Priorité: **24.07.78 FR 7821849**
**28.05.79 FR 7913532**

(43) Date de publication de la demande: **20.02.80**
**Bulletin 80/4**

(84) Etats contractants désignés: **AT DE IT NL SE**

(71) Demandeur: **Société Anonyme dite: HEXACHIMIE, 128, rue Danton, F-92500 Rueil-Malmaison (FR)**

(72) Inventeur: **Teulon, Jean-Marie, Charles, 12, Résidence du Bel Ebat 56, avenue de Verdun, F-78170 La Celle Saint Cloud (FR)**

(74) Mandataire: **de Haas, Michel et al, Cabinet Beau de Lomenie 55 rue d'Amsterdam, F-75008 Paris (FR)**

(54) **Nouveaux esters d'acide indane-acétique, leur préparation et médicaments les contenant.**

(57) Nouveaux composés de formule:

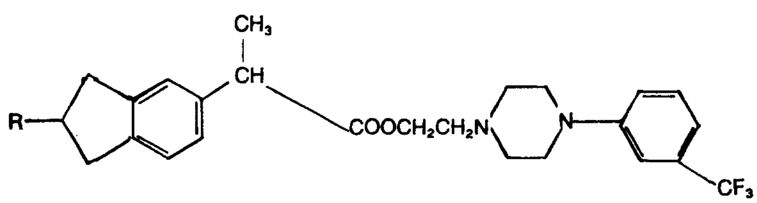

dans laquelle R représente un groupe éthyle ou isopropyle, et leurs sels d'addition d'acide ou d'ammonium quaternaire.

Préparation à partir des composés:

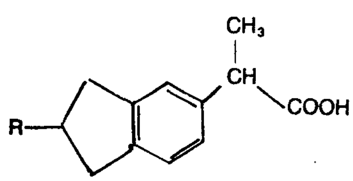

ou

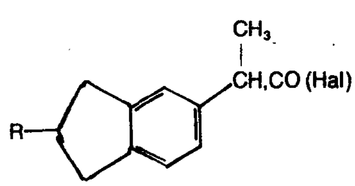

dans laquelle R a la même signification que ci-dessus.

Analgésiques.

EP 0 008 256 A2

TITRE MODIFIÉ
voir page de garde



Nouveaux esters aminés d'acide indane-acétique et leur utilisation en thérapeutique.

La présente invention concerne de nouveaux esters aminés de l'indane, leur procédé de préparation et leur application en thérapeutique.

Ces nouveaux dérivés répondent à la formule générale :

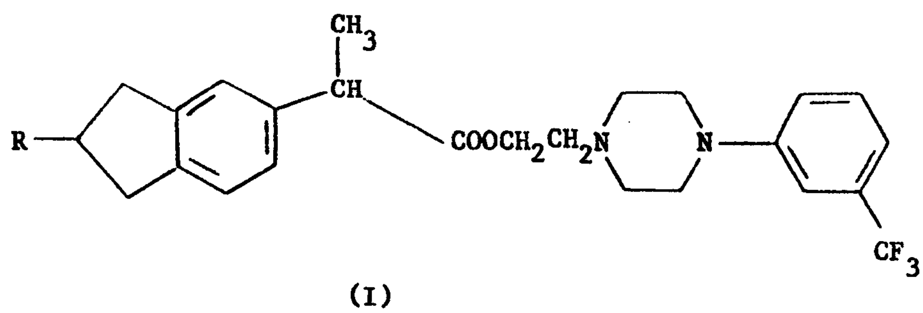

(I)

dans laquelle R est un groupe éthyle ou isopropyle.

L'invention vise également les sels d'addition des composés de formule I : les sels d'addition d'acides ou les sels d'ammonium quaternaire.

Les sels d'addition d'acides sont obtenus par réaction avec un acide minéral ou organique selon une méthode connue en soi.

Parmi les acides utilisables à cet effet, on peut notamment citer les acides chlorhydrique, sulfurique, phosphorique, oxalique, succinique, méthanesulfonique, cyclohexylsulfamique, formique, aspartique, glutamique, N-acétylaspartique, N-acétylglutamique, ascorbique, maléique, malique, fumarique, lactique, benzoïque, cinnamique, p-toluènesulfonique.

Les nouveaux composés selon l'invention sont doués d'activités pharmacologiques intéressantes et peuvent être utiles en thérapeutique comme agents analgésiques.

Selon l'invention, on propose des compositions thérapeutiques utiles notamment pour le traitement de la douleur caractérisées en ce qu'elles renferment, en association avec un excipient physiologiquement acceptable, une quantité efficace d'au moins un composé de formule I ou d'un de ses sels d'addition non toxiques.

Pour préparer un composé de formule I on dispose de plusieurs méthodes, applications de principes connus. Un procédé de préparation est le suivant :

a) on fait réagir les halogénures (III) des acides de formule II :

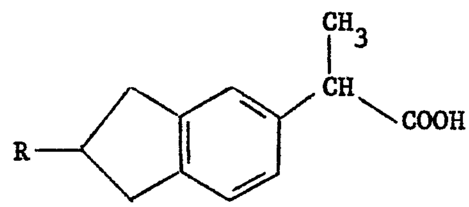

(II)

dans laquelle R a la même signification que ci-dessus, avec l'amino-alcool de formule :

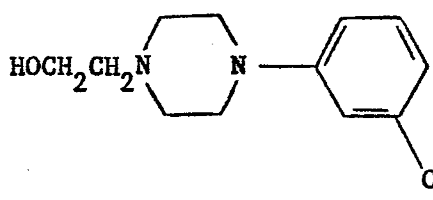

ou

b) on fait réagir les sels minéraux (IV) des acides de formule II avec un halogénure de formule :

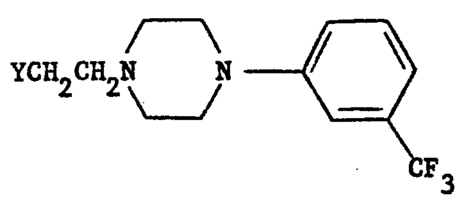

dans laquelle Y est un atome d'halogène.

Les étapes du procédé selon l'invention sont illustrées dans le schéma suivant :

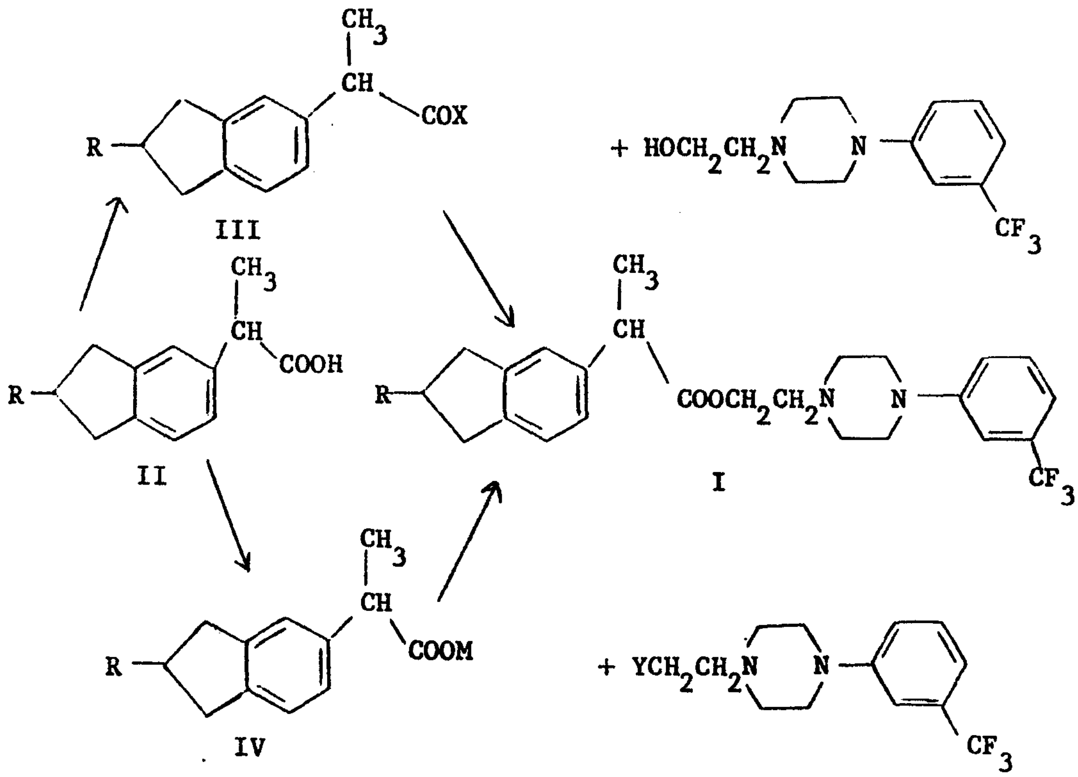

III II I IV

(X = atome d'halogène)

(M = métal)

Pour préparer les composés de formule I on peut aussi disposer de la méthode illustrée par le schéma suivant :

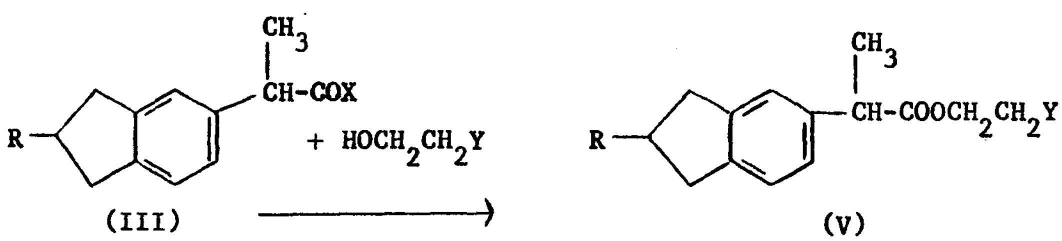

(III) (V)

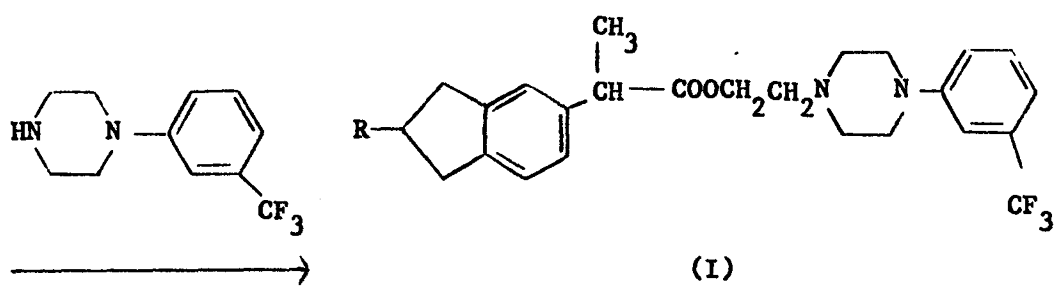

(I)

dans lequel :

R est un groupe éthyle ou isopropyle,

X et Y sont des atomes d'halogène.

a) on fait réagir les halogénures d'acide de formule III avec un halogéno-alcool $HOCH_2CH_2Y$ dans un solvant organique comme l'acétone, le chloroforme, le chlorure de méthylène ou un hydrocarbure aromatique en présence ou non d'une base comme la pyridine ou la triéthylamine.

b) on fait réagir l'ester halogéné obtenu de formule V avec la m-trifluorométhylphénylpipérazine:

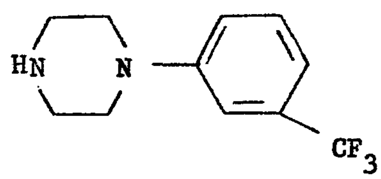

dans un solvant organique comme l'acétone, le chloroforme, le chlorure de méthylène ou un hydrocarbure aromatique en utilisant soit le double de m-trifluorométhylphényl pipérazine soit une base azotée tertiaire comme la triéthylamine.

L'halogénure de formule (V) est nouveau et fait partie de l'invention.

L'invention est illustrée ci-dessous par des exemples de synthèse non limitatifs.

Exemple 1.

Chlorure de l'acide méthyl-2[(éthyl-2)indanyl-5]acétique.

Une solution de 57 g d'acide méthyl-2[(éthyl-2) indanyl-5] acétique, 40 ml de chlorure de thionyle dans 200 ml de benzène est portée au reflux durant 2 heures. Le solvant et l'excès de chlorure de thionyle sont ensuite évaporés sous vide. L'huile obtenue est ensuite soumise à distillation sous vide. On récupère ainsi 56 g de chlorure de l'acide méthyl-2[(éthyl-2)indanyl-5]acétique sous forme d'un liquide.

$$Eb_{(1mmHg)} = 125\text{-}128°C.$$

Exemple 2.

Chlorhydrate de l'ester(m-trifluorométhylphényl)pipérazino éthylique de l'acide méthyl-2[(éthyl-2)indanyl-5]acétique.

A 64 g de chlorhydrate de (m-trifluorométhylphényl) pipérazino-éthanol dans 600 ml de benzène anhydre, on ajoute goutte à goutte 63 ml de triéthylamine.

On laisse agiter 30 min à température ambiante après la fin de l'addition puis on ajoute goutte à goutte 55,9 g de chlorure de l'acide méthyl-2[(éthyl-2)indanyl-5]acétique préparé à l'exemple 1 dans 100 ml de benzène.

Après la fin l'addition le mélange réactionnel est porté au reflux durant 5 heures.

Après refroidissement, la phase organique est soigneusement lavée à l'eau, avec une solution de soude 5% en présence de glace, puis encore à l'eau, séchée sur carbonate de sodium. Après évaporation du benzène, le résidu qui se présente sous forme d'un liquide est dissous dans l'acétone et additionné d'éther chlorhydrique jusqu'à pH acide. Les cristaux formés sont essorés et soigneusement lavés à l'acétone. On isole ainsi 70 g du chlorhydrate de l'ester(m-trifluorométhylphényl)pipérazino éthylique de l'acide méthyl-2[(éthyl-2)indanyl-5]acétique sous forme de cristaux blancs de point de fusion 199-201°C.

Exemple 3.

Chlorure de l'acide méthyl-2[(isopropyl-2)indanyl-5]acétique.

Selon le mode opératoire de l'exemple 1 mais en utilisant 31 g d'acide méthyl-2[(isopropyl-2)indanyl-5]acétique on récupère 30 g de chlorure de l'acide méthyl-2[(isopropyl-2) indanyl-5]acétique sous forme d'un liquide :

$$Eb_{(1\ mmHg)} = 150°C.$$

Exemple 4.

Ester(m-trifluorométhylphényl)pipérazino éthylique de l'acide méthyl-2[(isopropyl-2)indanyl-5]acétique.

Une solution de 6,9 g du chlorure de l'acide méthyl-2[(isopropyl-2)indanyl-5]acétique, préparé à l'exemple 3, dans 20 ml de benzène est ajoutée goutte à goutte à une solution de

7,5 g de (m-trifluorométhylphényl)pipérazino éthanol, 4,5 ml de triéthylamine dans 100 ml de benzène.

Après la fin de l'addition, le mélange réactionnel est porté au reflux durant 5 heures.

Après refroidissement, la phase organique est soigneusement lavée à l'eau, séchée sur carbonate de sodium et le benzène est évaporé sous vide. Le résidu obtenu est repris par du pentane à froid. Les cristaux formés sont essorés et lavés au pentane. On isole ainsi 11 g d'ester(m-trifluorométhylphényl)pipérazino éthylique de l'acide méthyl-2[(isopropyl-2)indanyl-5]acétique sous forme de cristaux blancs de point de fusion 56-58°C.

Exemple 5.

Chlorhydrate de l'ester(m-trifluorométhylphényl)pipérazino éthylique de l'acide méthyl-2[(éthyl-2)indanyl-5]acétique.

21,8 g de l'acide méthyl-2[(éthyl-2)indanyl-5)acétique sont traités par une solution de méthylate de sodium préparée à partir de 2,3 g de sodium dissous dans 40 ml de méthanol. Après évaporation du solvant, on obtient le sel de sodium de l'acide sous forme d'une poudre blanche.

On porte au reflux durant 7 heures une solution de ce sel de sodium et de 29,3 g de [(m-trifluorométhylphényl)pipérazino]β-chloroéthane dans 100 ml de xylène.

Après refroidissement du mélange réactionnel, la phase organique est lavée à l'eau et séchée sur carbonate de sodium. Après évaporation du solvant, on isole la base sous forme d'un liquide.

Cette base est reprise par de l'acétone et additionnée d'éther chlorhydrique jusqu'à pH acide. Les cristaux obtenus sont soigneusement lavés à l'acétone et séchés. On récupère ainsi 36 g de chlorhydrate de l'ester (m-trifluorométhyphényl)pipérazino éthylique de l'acide méthyl-2[(éthyl-2)indanyl-5]acétique sous forme de cristaux blancs de point de fusion 200-2°C.

Exemple 6.

Ester(m-trifluorométhylphényl)pipérazino éthylique de l'acide méthyl-2[(isopropyl-2)indanyl-5]acétique.

Selon le mode opératoire de l'exemple 5 mais en utilisant 23,2 g d'acide méthyl-2[(isopropyl-2)indanyl-5]acétique on récupère 37 g d'ester(m-trifluorométhylphényl)pipérazino éthylique de l'acide méthyl-2[(isopropyl-2)indanyl-5]acétique base sous forme de cristaux blancs de point de fusion 56-58°C.

Exemple 7.

Ester bromo éthylique de l'acide méthyl-2[(éthyl-2)indanyl-5]acétique.

formule V R = éthyle Y = Br

Méthode A :

On porte au reflux durant 4 heures la solution de 4,4 g de chlorure de l'acide méthyl-2[(éthyl-2)indanyl-5]acétique et 2,45 g de bromo-2 éthanol dans 50 ml d'acétone anhydre.

Après évaporation du solvant, le résidu huileux obtenu est dissous dans l'éther qu'on lave en présence de glace, avec une solution aqueuse de bicarbonate de sodium, à l'eau puis qu'on sèche sur sulfate de sodium. Après filtration et évaporation du solvant on obtient 5,9 g d'ester bromo éthylique de l'acide méthyl-2[(éthyl-2)indanyl-5]acétique sous forme d'un résidu huileux qu'on utilise brut pour l'étape suivante.

Méthode B :

On additionne à 0°C une solution de 12 g de chlorure de l'acide méthyl-2[(éthyl-2)indanyl-5]acétique dans 15 ml de chloroforme à une solution de 6,4 g de bromo-2 éthanol et 5,5 ml de pyridine dans 30 ml de chloroforme.

Après la fin de l'addition, on porte le mélange réactionnel au reflux durant 1 heure.

Après refroidissement, le mélange réactionnel est lavé à l'eau, avec de l'acide chlorhydrique 5% puis encore à l'eau. La phase chloroformique est séchée et après évaporation du solvant le résidu huileux est soumis à distillation sous vide. On récupère ainsi 13,7 g de l'ester bromo éthylique de l'acide méthyl-2[(éthyl-2)indanyl-5]acétique sous forme d'une huile.

$Eb_{0,2\ mm\ de\ Hg}$ = 138-148°C.

Exemple 8.

Chlorhydrate de l'ester m-trifluorométhylphénylpipérazino éthylique de l'acide méthyl-2[(éthyl-2)indanyl-5]acétique.

formule I R = éthyle

Méthode A :

A une solution de 4,2 g de m-trifluorométhylphénylpipérazine et de 3 ml de triéthylamine dans 25 ml d'acétone anhydre on ajoute une solution de 5,9 g d'ester bromo éthylique de l'acide méthyl-2[(éthyl-2)indanyl-5]acétique, obtenu par la méthode A de l'exemple 7, dans 15 ml d'acétone anhydre.

Le mélange réactionnel est agité 2 heures à température ambiante puis porté 6 heures au reflux.

Après refroidissement le bromhydrate de triéthylamine formé est essoré et le filtrat est concentré sous vide.

Le résidu obtenu est repris à l'éther et à nouveau refiltré.

Le nouveau filtrat récupéré est concentré sous vide. On obtient ainsi 9,8 g d'huile.

Cette huile est dissoute dans 50 ml d'acétone puis on additionne 25 ml d'eau et on acidifie à pH 3 avec de l'acide chlorhydrique concentré.

Les cristaux formés sont essorés, lavés à l'eau puis à l'acétone et séchés.

On récupère ainsi 2,7 g de chlorhydrate de l'ester m-trifluorométhylphényl pipérazino éthylique de l'acide méthyl-2 [(éthyl-2)indanyl-5]acétique sous forme de cristaux blancs de point de fusion 201-203°C.

Méthode B :

On porte au reflux durant 10 heures une solution de 13,6 g de l'ester bromo éthylique de l'acide méthyl-2[(éthyl-2) indanyl-5]acétique, préparé à l'exemple 7 par la méthode B, 9,6 g de m-trifluorométhylphénylpipérazine, 7 ml de triéthylamine et 200 mg d'iodure de sodium dans 100 ml de benzène anhydre.

On refroidit le mélange réactionnel, on le lave soigneusement à l'eau, on le sèche sur sulfate de sodium et on évapore le benzène sous vide.

Le résidu huileux obtenu : 20 g est repris par 60 ml d'acétone et on ajoute à froid 3,5 ml d'acide chlorhydrique concentré puis 40 ml d'eau.

Les cristaux formés sont essorés, lavés avec une faible quantité d'eau puis à l'écétone et séchés.

On récupère ainsi 14,2 g de chlorhydrate de l'ester m-trifluorométhylphénylpipérazino éthylique de l'acide méthyl-2 [(éthyl-2)indanyl-5]acétique sous forme de cristaux blancs de point de fusion 202-203°C.

Exemple 9.

Ester bromo éthylique de l'acide méthyl-2[(isopropyl-2)indanyl-5] acétique.

formule V R = isopropyle Y = Br

Méthode A :

On additionne à 0°C une solution de 32 g de chlorure de l'acide méthyl-2[(isopropyl-2)indanyl-5]acétique dans 25 ml de chloroforme à une solution de 16 g de bromo-2 éthanol et 14 ml de pyridine dans 50 ml de chloroforme.

Après la fin de l'addition, on porte le mélange réactionnel au reflux durant 1 heure.

Après refroidissement le mélange réactionnel est lavé à l'eau, avec de l'acide chlorhydrique 5% puis encore à l'eau.

La phase chloroformique est séchée et après évaporation du solvant le résidu huileux 49 g est soumis à distillation sous vide. On récupère ainsi 35,2 g de l'ester bromo éthylique de l'acide méthyl-2[(isopropyl-2)indanyl-5]acétique sous forme d'une huile.

$Eb_{1,5\ mm\ Hg} = 165\text{-}175°C.$

Méthode B :

On additionne goutte à goutte une solution de 32 g de chlorure de l'acide méthyl-2[(isopropyl-2)indanyl-5]acétique dans 25 ml d'acétone à une solution de 16 g de bromo-2 éthanol et 10,4 ml de pyridine dans 125 ml d'acétone.

Après la fin de l'addition, on porte le mélange réactionnel au reflux durant 1 h 30.

Le mélange réactionnel est ensuite concentré sous vide, repris à l'éther qu'on lave à l'eau, avec de l'acide chlorhydrique 5% puis encore à l'eau. La phase éthérée séchée, le solvant est évaporé sous vide.

On récupère ainsi 40 g de l'ester bromo éthylique de l'acide méthyl-2[(isopropyl-2)indanyl-5]acétique sous forme d'une huile que l'on utilise brute pour la suite des opérations.

Exemple 10.

Chlorhydrate de l'ester m-trifluorométhylphénylpipérazino éthylique de l'acide méthyl-2[(isopropyl-2)indanyl-5]acétique.

formule I R = isopropyle

Méthode A :

On porte au reflux durant 8 heures une solution de 35,2 g de l'ester bromo éthylique de l'acide méthyl-2[(isopropyl-2)indanyl-5]acétique préparé à l'exemple 9 par la méthode A, 47,8 g de m-trifluorométhylphénylpipérazine et de 1 g d'iodure de sodium dans 300 ml de toluène anhydre.

On refroidit le mélange réactionnel et on essore le bromhydrate de m-trifluorométhylphénylpipérazine qu'on lave au benzène.

Le filtrat organique est lavé à l'eau puis séché et concentré sous vide.

Le résidu huileux obtenu de 61,7 g est repris par 250 ml d'acétone et on ajoute à froid 8,7 ml d'acide chlorhydrique concentré puis 150 ml d'eau.

Les cristaux obtenus sont essorés, lavés avec une faible quantité d'eau puis de l'acétone et séchés.

On récupère ainsi 35,7 g de chlorhydrate de l'ester m-trifluorométhylphénylpipérazino éthylique de l'acide méthyl-2 [(isopropyl-2)indanyl-5]acétique sous forme de cristaux blancs de point de fusion 191-193°C.

Méthode B :

On porte au reflux durant 8 heures une solution de

40 g de l'ester bromo éthylique de l'acide méthyl-2[(isopropyl-2) indanyl-5]acétique, préparé à l'exemple 9 par la méthode B, 31,5 g de chlorhydrate de m-trifluorométhylphénylpipérazine et de 36,5 ml de triéthylamine dans 200 ml d'acétone.

Le mélange réactionnel est ensuite concentré sous vide, repris par un mélange d'eau et de glace et extrait à l'éther. La phase éthérée lavée à l'eau est séchée sur sulfate de sodium puis concentrée sous vide.

Le résidu obtenu 57 g est repris par 150 ml d'acétone, additionné de 10 ml d'acide chlorhydrique concentré puis de 100 ml d'eau. Les cristaux formés sont essorés, lavés avec une faible quantité d'eau puis de l'acétone et séchés.

On récupère ainsi 27,4 g de chlorhydrate de l'ester m-trifluorométhylphénylpipérazino éthylique de l'acide méthyl-2 [(isopropyl-2)indanyl-5]acétique sous forme de cristaux blancs de point de fusion 193-194°C.

Méthode C :

On additionne goutte à goutte une solution de 32 g de chlorure de l'acide méthyl-2[(isopropyl-2)indanyl-5]acétique dans 25 ml de chloroforme à une solution de 16 g de bromo-2 éthanol et 14 ml de pyridine dans 50 ml de chloroforme en refroidissant par de la glace.

Après la fin de l'addition on laisse le mélange réactionnel revenir à température ambiante puis on porte 1 heure au reflux.

On laisse ensuite le mélange réactionnel revenir à température ambiante et on rajoute 19,6 ml de triéthylamine et 29,5 g de m-trifluorométhylphénylpipérazine.

La réaction est ensuite portée au reflux durant 12 h.

Après refroidissement on dilue avec du chloroforme et on lave soigneusement à l'eau.

La phase chloroformique est séchée et le solvant est évaporé sous vide.

Le résidu obtenu : 50,7 g est repris par 150 ml d'acétone puis additionné de 8,5 ml d'acide chlorhydrique concentré et 100 ml d'eau.

Les cristaux obtenus sont essorés, lavés avec une faible quantité d'eau puis avec de l'acétone et séchés.

On récupère ainsi 27,3 g de chlorhydrate de l'ester m-trifluorométhylphénylpipérazino éthylique de l'acide méthyl-2 [(isopropyl-2)indanyl-5]acétique sous forme de cristaux blancs de point de fusion 193-194°C.

On trouvera ci-dessous des résultats pharmocologiques concernant les deux nouveaux esters aminés de l'indane, de formule I, selon l'invention, mettant en évidence :

A) leur activité analgésique :

Des lots de 12 souris mâle (SPF souche $OF_1$) pesant 19-20g reçoivent les produits essayés, par voie orale (VO). Une heure après, on injecte par voie intrapéritonéale 0,3 ml/souris d'une solution à 0,02% de phénylbenzoquinone, et de la 5ème à la 10ème minute après ce dernier traitement, on compte le nombre de réactions douloureuses (torsions abdominales).

Le tableau ci-après donne le pourcentage d'inhibition de ces réactions.

| $mg.kg^{-1}$ VO | Exemples 2 ou 5 | Exemples 4 ou 6 |
|---|---|---|
| 1 | 22 | |
| 2 | 47 | |
| 4 | 59 | |
| 8 | 64 | 23 |
| 16 | 65 | 40 |
| 32 | 82 | 67 |
| 64 | | 75 |
| 128 | | 75 |
| $DE_{50}$ $mg.kg^{-1}$ VO | 4 | 25 |

B) leur toxicité :

Chez la souris, aucune mortalité n'a été observée, après administration intrapéritonéale jusqu'aux doses de :

512 $mg.kg^{-1}$ pour les exemples 2 ou 5

256 $mg.kg^{-1}$ pour les exemples 4 ou 6

Les composés (I) selon l'invention possèdent donc des propriétés analgésiques et sont très peu toxiques.

Ils peuvent donc être utilisés en thérapeutique humaine pour soulager les douleurs aiguës et chroniques de diverses origines, sous forme de comprimés ou de gélules dosés de 100 à 200 mg, de suppositoires dosés de 300 à 500 mg. La posologie quotidienne peut être de 0,5 à 2 g environ d'ingrédient actif, pour un adulte.

# REVENDICATIONS

1. Nouveaux composés, caractérisés en ce qu'ils répondent à la formule (I) :

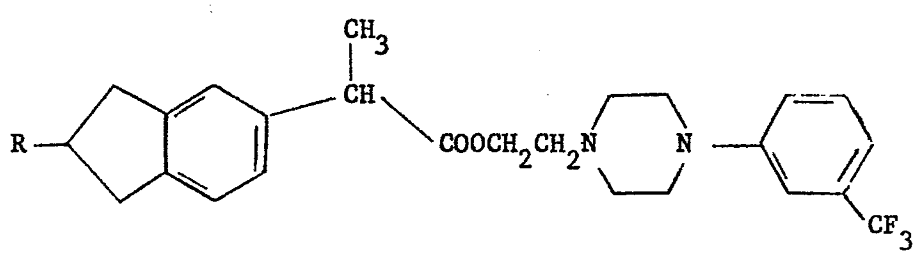

(I)

dans laquelle R représente un groupe éthyle ou isopropyle, et leurs sels d'addition d'acide ou d'ammonium quaternaire, notamment les chlorhydrates.

2. Procédé de préparation des composés selon la revendication 1, caractérisé en ce que l'on transforme l'acide (II) :

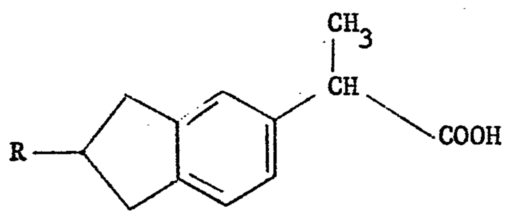

(II)

dans lequel R est tel que défini dans la revendication 1, en un halogénure (III) :

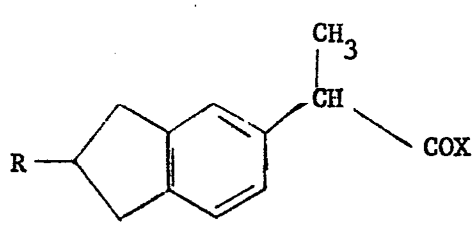

(III)

dans lequel X représente un atome d'halogéne, après quoi on fait réagir cet halogénure (III) sur un aminoalcool de formule :

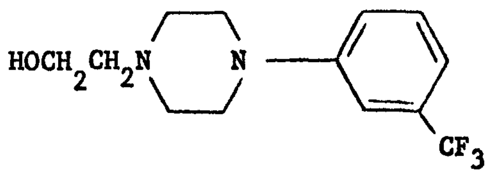

3. Procédé de préparation des composés selon la revendication 1, caractérisé en ce que l'on transforme l'acide (II) :

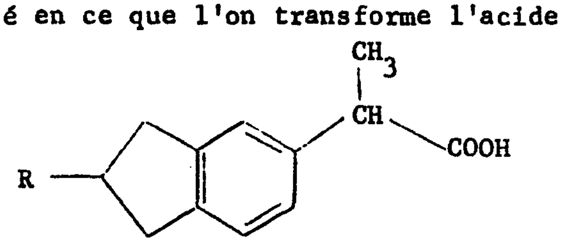

(II)

dans lequel R est tel que défini dans la revendication 1, en un sel de formule IV :

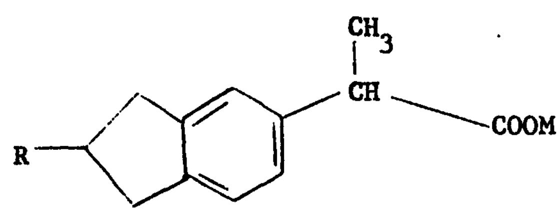

(IV)

dans laquelle M représente un métal, de préférence le sodium, après quoi on fait réagir ce sel sur un halogénure de formule :

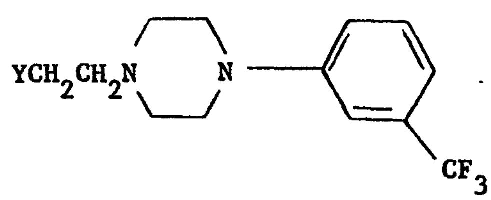

dans laquelle Y représente un atome d'halogène.

4. Procédé de préparation des composés selon la revendication 1, caractérisé en ce que l'on fait réagir un composé de formule :

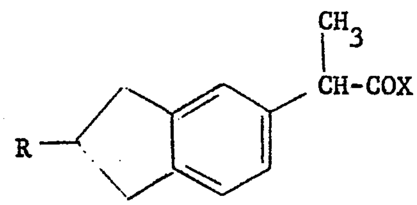

(III)

sur un halogénoalcool $OHCH_2CH_2Y$ dans un solvant organique pour obtenir l'ester halogéné de formule :

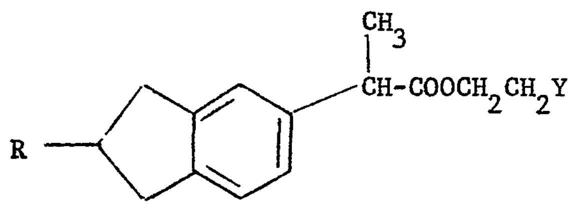

(V)

que l'on fait réagir sur la m-trifluorométhylphénylpipérazine de formule :

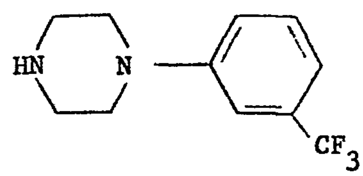

dans un solvant organique, R étant un groupe éthyle ou isopropyle et X et Y un atome d'halogène.

5. Procédé selon la revendication 4, caractérisé en ce que le solvant de la première étape est choisi parmi l'acétone, le chloroforme, le chlorure de méthylène ou un hydrocarbure aromatique.

6. Procédé selon la revendication 4 ou 5, caractérisé en ce que l'on effectue la première étape en présence d'une base comme la pyridine ou la triéthylamine.

7. Procédé selon l'une quelconque des revendications 4 à 6, caractérisé en ce que le solvant de la seconde étape est choisi parmi l'acétone, le chloroforme, le chlorure de méthylène ou un hydrocarbure aromatique.

8. Procédé selon l'une quelconque des revendications 4 à 7, caractérisé en ce que l'on effectue la seconde étape en utilisant le double de m-trifluorométhylphénylpipérazine.

9. Procédé selon l'une quelconque des revendications 4 à 7, caractérisé en ce que l'on effectue la seconde étape en utilisant également une base azotée tertiaire comme la triéthylamine.

10. Nouveaux médicaments utiles notamment comme analgésiques, caractérisés en ce qu'ils comportent au moins un des nouveaux composés selon la revendication 1, ou leurs sels d'addition d'acide ou d'ammonium quaternaire non toxiques.

11. Nouveaux médicaments selon la revendication 10, conditionnés sous forme de comprimés ou de gélules dosés de 100 à 200 mg, de suppositoires dosés de 300 à 500 mg, une dose journalière étant d'environ 0,5 à 2 g d'ingrédient actif pour un adulte.

12. Nouveau composé, caractérisé en ce qu'il répond à la formule :

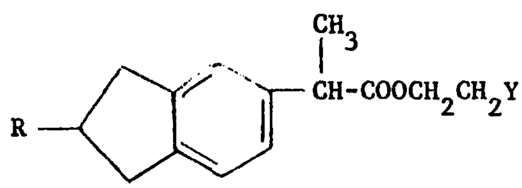

(V)

dans laquelle R et Y sont tels que définis ci-dessus dans la revendication 4.